# EUROPEAN PATENT APPLICATION

(11) **EP 4 261 331 A1**
(43) Date of publication of application: **18.10.2023**
(21) Application number: 23153087.4
(22) Date of filing: 24.01.2023
(51) Int. Cl.: D01H 13/16

(54) **A TWISTING UNIT WITH A STAND-ALONE THREAD BREAKAGE CONTROL SENSOR AND A METHOD FOR DETECTING THE BREAKAGE OF A THREAD BEING TWISTED**

(30) Priority: 30.03.2022 IT 202200006281
(71) Applicant: Savio Macchine Tessili S.p.A., 33170 Pordenone (IT)
(72) Inventor: SALA, Riccardo, I-33170 PORDENONE (IT); NICODEMO, Ruggero, I-33170 PORDENONE (IT); AL HOSEN, Kassem, I-33170 PORDENONE (IT)
(74) Representative: Mitola, Marco

(57) **Abstract**

Twisting unit (4) of a yarn (8) consisting of at least two threads which are twisted comprising at least one pigtail (12) thread guide inside which the yarn (8) twists along a longitudinal direction (X-X), cutting means of the yarn (8) configured to cut the yarn (8) in the event of detection of a breakage of at least one thread forming the yarn (8), at least one device for detecting the breakage (16) of at least one of said threads forming the yarn (8), comprising a tension sensor (20) of said yarn (8), a processing and control unit (24) operatively connected to said tension sensor (20) of said yarn (8). Advantageously the processing and control unit (24) is programmed so that upon starting and/or restoring the twisting process, a self-learning step is performed in which a reference value (R) of the tension of the yarn (8) is stored, to be monitored during twisting; if, during twisting, the tension of the yarn (8) undergoes a percentage decrease, with respect to said reference value (R), greater than or equal to a predefined maximum percentage deviation, the twisting is interrupted.

## Description

### FIELD OF APPLICATION

The present invention relates to a twisting unit with a stand-alone thread breakage control sensor and a method for detecting the breakage of a thread being twisted.

### STATE OF THE ART

As is known, in twisting machines, in particular of the duo-pot type, two or more strands of thread are coupled and joined by applying a mutual twist, so as to obtain a resulting yarn with superior technological features, in particular greater strength and regularity.

The twisted yarn, which exits from the twisting spindle, is passed inside a thread guide ring, usually called a pigtail, which conveys it towards the upper collection area, where it is wound on a tube so as to form a spool.

The pigtail plays a very important role in guiding the path of the thread and in adjusting the height of the balloon to contain the tension and change the shape thereof.

It can sometimes occur that, during the unravelling step, one or more threads forming the twisted thread break unintentionally and that, in the absence of a dedicated system for detecting the breakage of the thread, the winding in the spool continues without interruption, with the remaining threads being fed, while the broken thread begins to generate accumulations and tangles, making it particularly difficult to restore the functionality of the head.

In this regard, electronic systems are used for the continuous monitoring of the tension of the twisted thread, which represents the quantitative parameter most indicative of its goodness and regularity.

This is due to the fact that the possible breakage of one of the threads which contributes to the formation of the twisted thread induces a variation of the resistant section which immediately expresses a fluctuation of tension of the thread itself.

For example, the known systems aimed at solving these problems envisage measuring moment by moment the pressure exerted by the thread in the direction transverse to its stroke, this being directly related to its tension.

For example, the known art consists of the use of a piezoelectric sensor, commonly installed in direct proximity to the pigtail, provided with an open fork in ceramic material which receives a certain pressure in a transverse direction from the longitudinally running thread.

Since the contact pressure is proportional to the tension of the threads which have already been twisted and wound, a variation in tension due to a breakage modifies the pressure, and thus also the voltage of the output signal.

Typically, the control unit processes the output of the sensor to extrapolate information on the average yarn tension and the rotational speed of the balloon, starting from the amplitude and frequency of the signal, respectively.

The systems pertaining to the prior art are not free from disadvantages and drawbacks.

For example, patent EP0616058B1 describes a piezoelectric system for yarn quality control, in which the average tension and speed values deriving from the processing of the output signal of the piezoelectric sensor are compared by special comparators with reference threshold values, which must be entered manually by the operator in the machine PC.

Therefore, such values must be known a priori based on the features of the yarn to be processed, consequently undergoing changes at each batch change, to the detriment of the operating versatility of the machine.

Patent IT1239766B also discloses a piezoelectric device for monitoring thread tension, in which the response of the pressure sensor strongly depends on its installation position, since the contact pressure increases with the deviation imposed on the thread with respect to its nominal path.

Therefore, in the devices of the prior art the installation position of the sensor becomes extremely relevant, and must be reasonably chosen a priori according to the expected tension of the thread.

### PRESENTATION OF THE INVENTION

The need is therefore felt to resolve the drawbacks and limitations mentioned with reference to the prior art.

Such a need is met by a winding unit according to claim 1 and by a detection method according to claim 10.

### DESCRIPTION OF THE DRAWINGS

Further features and advantages of the present invention will be more clearly comprehensible from the description given below of preferred and non-limiting embodiments thereof, in which:

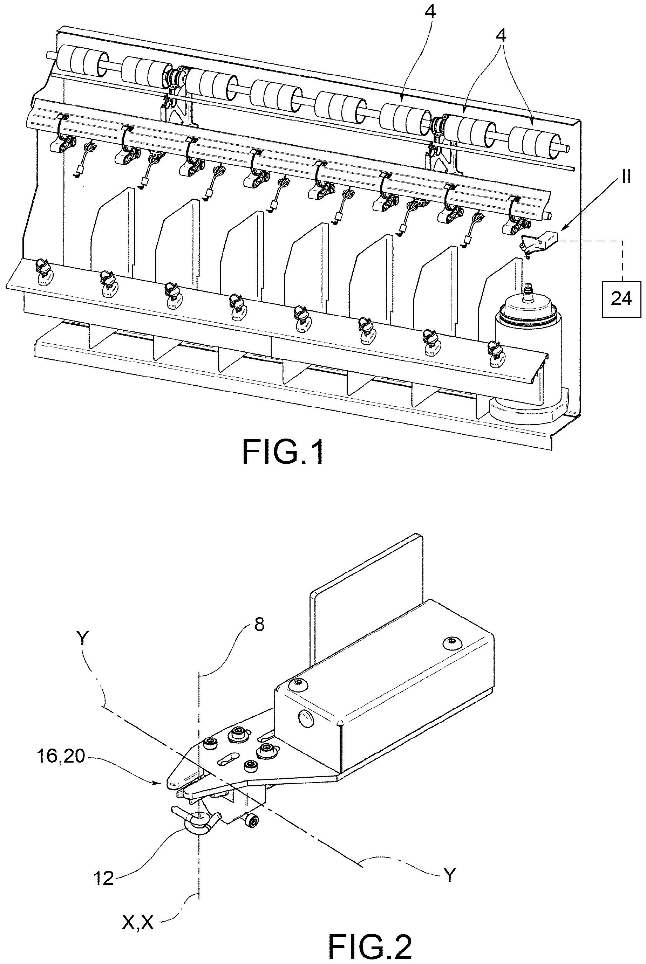

figure 1 depicts a perspective view of a twisting machine comprising a plurality of twisting units in accordance with the present invention;

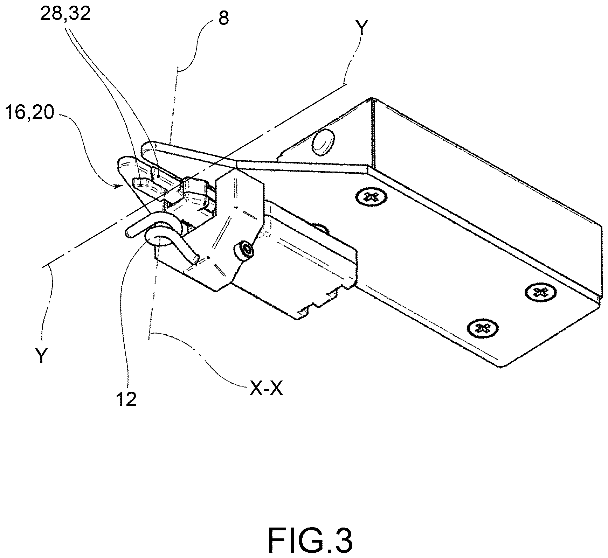

figures 2-3 depict perspective views, respectively from above and from below, of detail II of figure 1;
figure 4 depicts a flow-chart diagramming the operation of a twisting unit in accordance with an embodiment of the present invention.

The elements or parts of elements common to the embodiments described below will be indicated using the same reference numerals.

### DETAILED DESCRIPTION

With reference to the aforesaid figures, the number 4 globally indicates a twisting unit of a yarn 8 consisting of at least two threads which are twisted.

The twisting unit 4 comprises at least one pigtail 12 thread guide inside which the yarn 8 twists along a longitudinal direction X-X, and yarn cutting means (not depicted) configured to cut the yarn 8 in the event of detection of a breakage of at least one thread forming it.

The twisting unit 4 further comprises at least one device for detecting the breakage 16 of at least one of said threads forming the yarn 8, comprising a tension sensor 20 of said yarn 8.

The tension sensor 20 of the yarn 8, as better described below, is configured to measure, directly or indirectly, the tension to which the yarn 8 is subjected.

The tension sensor 20 of the yarn 8 comprises a processing and control unit 24 operatively connected to the tension sensor 20 of said yarn 8 itself.

In accordance with a possible embodiment, the tension sensor 20 is of the piezoelectric type.

The tension sensor 20 can be either of absolute type, in which the output signal is directly proportional to the input signal, or of relative type, in which generally the output is not directly proportional to the input.

In accordance with a possible embodiment, said tension sensor 20 comprises a fork element 28 within which the yarn 8 flows, said fork element 28 having two tines 32, substantially perpendicular to said longitudinal direction X-X.

Said tension sensor 20 is configured to measure the tension exerted by the yarn on at least one of said tines 32 along a transverse direction Y-Y, substantially perpendicular to said longitudinal direction X-X. Such tension in the transverse direction Y-Y is in turn a function of the longitudinal tension or traction of the yarn 8. In this case it is an indirect measurement of the traction of the yarn 8.

Advantageously, the processing and control unit 24 is programmed so that:
upon starting and/or restoring the twisting process, a self-learning step is performed in which a reference value R of the tension of the yarn 8 is stored, to be monitored during twisting,
if, during twisting, the tension of the yarn 8 undergoes a percentage decrease, with respect to said reference value R, greater than or equal to a predefined maximum percentage deviation, the twisting is interrupted.

Usually, the processing and control unit 24 is programmed to control the cutting means of the yarn 8 when a breakage is detected: in other words, after detecting a breakage, the yarn 8 is cut and then the twisting step is resumed.

Preferably, said reference value R of the tension of the yarn 8 is stored when it has stabilized in frequency within maximum oscillations less than 4 Hz.

For example, said reference value R of the tension of the yarn 8 is stored when the twisting spindle reaches a regime rotation speed, and consequently its signal has stabilized in frequency.

In accordance with a possible embodiment, the reference value R of the tension of the yarn 8 is a direct measurement value of said tension of the yarn 8.

It is also possible to envisage that the reference value R of the tension of the yarn 8 is an indirect measurement value of said tension, proportional to the tension of the yarn 8 to be monitored.

Preferably, said predefined maximum percentage difference is between 5% and 30%, depending on the total twists imparted to the yarn 8 and the type and number of twists of the individual threads forming the twisted yarn 8.

Such a value of the predefined maximum percentage difference can depend on the operating conditions of the twisting unit 4 and/or on the type of yarn 8 used; the type in turn can comprise the material of the yarn, its title, but also the number of threads forming it and the type of twisting to which they are subjected.

The processing and control unit 24 can also be programmed so as to use the tension sensor 20 as a thread presence sensor. This thereby avoids the additional use of the presence sensor of the yarn 8.

The functioning of the twisting unit in accordance with the present invention will now be described.

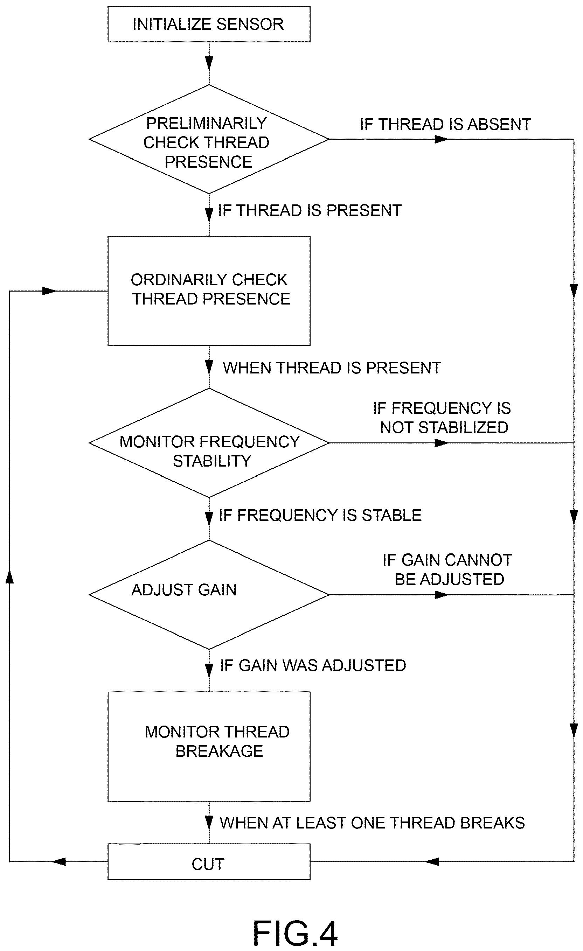

In particular (figure 4), when the unit is started, the tension sensor 20 is initialized.

A preliminary check for the presence of yarn 8 is then performed, for which:
if the yarn 8 is absent, a cut is commanded;
if the yarn 8 is present, the next step is proceeded to.

Thus an ordinary yarn presence check is performed, so that, when the yarn 8 is present, the signal frequency stability monitoring is started:
if the frequency has not stabilized, the yarn 8 is cut;
if the frequency is stable, the next step is proceeded to.

In such a next step, the signal gain adjustment is started:
if the gain cannot be adjusted, the yarn 8 is cut;
if the gain has been adjusted, the next step is proceeded to.

In such a further step, the monitoring of the breakage of at least one thread forming the twisted yarn is started.

When a breakage occurs, the tension sensor detects that the tension of the yarn 8 undergoes a decrease; in particular, if such a percentage decrease, with respect to said reference value R, is greater than or equal to a maximum predefined percentage deviation, the twisting is interrupted.

The yarn 8 is then cut and the ordinary presence control step of the yarn 8 is returned to, then repeating the cycle just described when the presence of the yarn 8 is detected again.

As can be appreciated from what has been described, the present invention has considerable advantages over the current solutions of the prior art and makes it possible to overcome the drawbacks thereof.

Firstly, by virtue of the use of a self-adaptive sensor, the operator no longer has to be concerned with entering any reference value of the thread tension in the PC (manually), as a function of the specific fibre or title to be processed or the linear speed of yarn collection.

The unit is perfectly capable of setting itself at start-up or upon restarting, and independently managing the detection of a thread breakage, stopping the twisting and the subsequent restoration of the twisting itself.

Furthermore, the tension sensor thus configured can also be used as a thread presence sensor, helping to simplify the mechanical and electronic architecture of the twisting unit and consequently reduce the dimensions thereof.

Furthermore, in the event that a piezoelectric voltage sensor is used, once the self-learning procedure has been performed, the installation position of the tension sensor itself becomes irrelevant, because what is monitored is a relative (and not absolute) deviation or shift of the tension signal, without there being any need to make the value detected coincide with the set-point set by prior adjustment of the tension sensor itself (as is instead the case in the solutions of the prior art).

A person skilled in the art may make numerous modifications and variations to the solutions described above so as to satisfy contingent and specific requirements.

The scope of protection of the present invention is defined by the following claims.

## Claims

1. Twisting unit (4) of a yarn (8) consisting of at least two threads which are twisted comprising:
- at least one pigtail (12) thread guide inside which the yarn (8) twists along a longitudinal direction (X-X),
- cutting means of the yarn (8) configured to cut the yarn (8) in the event of detection of a breakage of at least one thread forming the yarn (8),
- at least one device for detecting the breakage (16) of at least one of said threads forming the yarn (8), comprising a tension sensor (20) of said yarn (8),
- a processing and control unit (24) operatively connected to said tension sensor (20) of said yarn (8), **characterized in that**
the processing and control unit (24) is programmed so that:
- upon starting and/or restoring the twisting process, a self-learning step is performed in which a reference value (R) of the tension of the yarn (8) is stored, to be monitored during twisting,
- if, during twisting, the tension of the yarn (8) undergoes a percentage decrease, with respect to said reference value (R), greater than or equal to a predefined maximum percentage deviation, the twisting is interrupted.

2. Twisting unit (4) according to claim 1, wherein said reference value (R) of the tension of the yarn (8) is stored when it has stabilized in frequency.

3. Twisting unit (4) according to claim 2, wherein said reference value (R) of the tension of the yarn (8) is stored when it has stabilized in frequency within maximum oscillations less than 4 Hz.

4. Twisting unit (4) according to claim 1, 2 or 3, wherein said reference value (R) of the tension of the yarn (8) is stored when the twisting spindle reaches a regime rotation speed.

5. Twisting unit (4) according to claim 1, 2, 3 or 4, wherein the reference value (R) of the tension of the yarn (8) is a direct measurement value of said tension of the yarn (8).

6. Twisting unit (4) according to claim 1, 2, 3 or 4, wherein the reference value (R) of the yarn tension is an indirect measurement value of said tension, proportional to the tension of the yarn (8) to be monitored.

7. Twisting unit (4) according to any one of claims 1 to 6, wherein said predefined maximum percentage difference is between 5% and 30%.

8. Twisting unit (4) according to any one of claims 1 to 7, wherein the processing and control unit (24) is programmed so as to use the tension sensor (20) as a presence sensor of the yarn (8).

9. Twisting unit (4) according to any one of claims 1 to 8, wherein said tension sensor (20) comprises a fork element (28) within which the yarn (8) flows, said fork element (28) having two tines (32), substantially perpendicular to said longitudinal direction (X-X), wherein said tension sensor (20) measures the tension exerted by the yarn (8) on at least one of said times (32) along a transverse direction (Y-Y) substantially perpendicular to said longitudinal direction (X-X).

10. Method for detecting the breakage of at least one of the twisted threads forming a yarn (8), in a twisting unit (4), comprising the steps of:
- upon starting and/or restoring the twisting process, performing a self-learning step in which a reference value (R) of the tension of the yarn (8) is stored, to be monitored during twisting,
- if, during twisting, the tension of the yarn (8) undergoes a percentage decrease, with respect to said reference value (R), greater than or equal to a predefined maximum percentage deviation, interrupting the twisting.

11. Method according to claim 10, comprising the step of storing said reference value (R) of the tension of the yarn (8) when it has stabilized in frequency.

12. Method according to claim 11, wherein said reference value (R) of the tension of the yarn (8) is stored when it has stabilized in frequency within maximum oscillations less than 4 Hz.

13. Method according to claim 10, 11 or 12, comprising the step of storing said reference value (R) of the tension of the yarn (8) when the twisting spindle reaches a regime rotation speed.

14. Method according to claim 10, 11, 12 or 13, comprising the step of directly measuring the reference value (R) of the tension of the yarn (8).

15. Method according to claim 10, 11, 12 or 13, comprising the step of indirectly measuring the reference value (R) of the tension of the yarn (8), by measuring a signal proportional to the tension of the yarn (8) to be monitored.

16. Method according to any one of claims 10 to 15, wherein said predefined maximum percentage difference is between 5% and 30%.
